# EUROPEAN PATENT APPLICATION

(11) **EP 0 763 352 A1**
(43) Date of publication of application: **19.03.1997**
(21) Application number: 96830466.7
(22) Date of filing: 11.09.1996
(51) Int. Cl.: A61F 5/455

(54) **Sanitary canaliser to urinate, particularly for the female urination in public restrooms**

(30) Priority: 14.09.1995 IT RM950614
(71) Applicant: Bertolani, Sergio, 00069 Morlupo, Roma (IT)
(72) Inventor: Bertolani, Sergio, 00069 Morlupo, Roma (IT)
(74) Representative: Iannone, Carlo Luigi

(57) **Abstract**

The invention relates to a sanitary canaliser (1) to urinate, particularly for the female urination in public restrooms substantially made up of a sheet element having a main body (2), foldable along a substantially central line (3) and the lateral edges of which are shaped in such a way to make him assuming a substantially frusto conical shape when it is folded.

## Description

The present invention relates to a sanitary canaliser, particularly for the female urination in public restrooms.

More particularly, the invention concerns a device of the above kind particularly suitable for the solution of the problems deriving from the use of public restrooms.

The solution proposed according to the invention is particularly useful when one, who is out of his house, during a trip, on a train, on an aeroplane, in a motorway restaurant or snack bar, is obliged to use public restrooms, the lack of hygiene of which is well known as well as are know the difficulties for a woman in these cases to try to avoid the direct contact of her skin with the board of the water-closet, when it is provided, or, in case it lacks, directly on the edge of the water-closet or of any other part of the surrounding environment.

Obviously, the solution suggested according to the present invention can be used also in different situations, for example for incontinent male subjects.

Everybody knows the drawbacks for the women connected with the needing of urinating in public restrooms, being it necessary to move near the most delicate and most subjected to infection part of the female body to dirty and infect surfaces, i.e. to the water-closet and to the relevant board.

A well evident example of this fact are the restrooms of the trains, of the aeroplanes, of the motorway restaurants or snack bars, and of other king of public rooms or premises.

In the field of the hygiene for water-closets, besides the disinfectants, the liquid or spray detergents, only one solution exists, which comprises a paper or plastic sheet having a shape conforming to the shape of the water-closet board upon which said sheet must be put in order to avoid any contact between the skin and the board, thus difficulty obtaining a practical result.

In this context the solution according to the invention is included, said solution allowing to solve in a practical, simple and cheap way all the above mentioned drawbacks.

These and other results are obtained according to the invention by a canaliser, mainly made up of paper material, having substantially a frusto conical shape, suitably stiffen, aseptic, preferably disposable, suitable to receive and canalise the urine of a woman, allowing to the same to stay in an upright position during the miction, avoiding in such a way any dangerous contact with the water-closet.

This kind of solution allows to obtain a remarkable ease of use, a sterile product, mainly if it is not completely disposable, easy to be brought and safe since it is characterized by an anatomical shape allowing to the same to perfectly adhere to the vulva, receiving within the ellipse of the base of the frusto conic element, the labia majora and the labia minora, the clitoris and then the outer orificium of the urethra, allowing a regular urination without lateral leakage of the liquid.

It is therefore specific object of the present invention a sanitary canaliser to urinate, particularly for the female urination in public restrooms substantially made up of a sheet element having a main body, foldable along a substantially central line and the lateral edges of which are shaped in such a way to make him assuming a substantially frusto conical shape when it is folded.

Preferably, according to the invention, on one of the lateral edges of said main body a closure foldable flap can be provided.

Particularly, the canaliser according to the invention can be made up of plasticized or waxed paper material.

Furthermore, according to the invention, said lateral edges of said main body provide each one a first diverging length and a second converging length.

Always according to the invention, on said lateral edges of the main body further flap for the coupling of a crown element can be provided, said crown element being preferably comprised of soft, sterile and absorbing material.

The inner part of the surface of the canaliser according to the invention can be coated by a perfumed absorbing material or by a medicated material in case of local pathologies.

Always according to the invention, said canaliser can be comprised of two separated parts, the outer one of which can be used again and the inner one of which is soft and disposable.

The present invention will be now described, for illustrative but not limitative purposes, according to its preferred embodiments, with particular reference to the figures of the enclosed drawings, wherein:
figure 1 is cross-section view of an embodiment of the canaliser according to the invention;
figure 2 is a plan view of the canaliser of figure 1;
figures 3a and 3b respectively show the canaliser of figure 1 closed and ready to use; and
figure 4 is a top view of the canaliser of figure 1 provided with a further crown element.

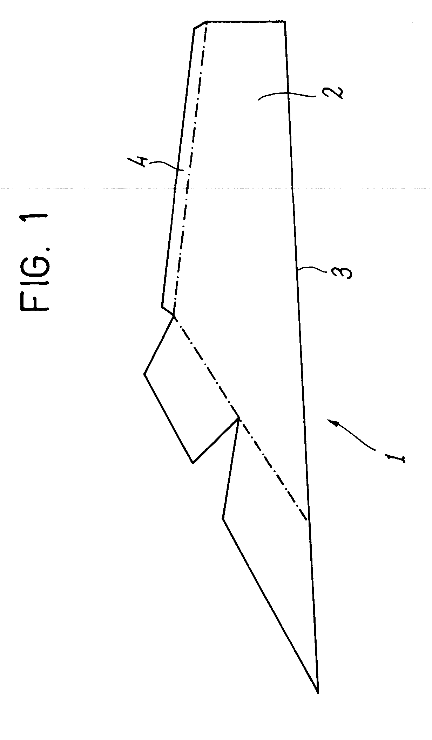

Making at first reference to figures 1 - 3, an embodiment of the canaliser 1 according to the invention is shown.

Said canaliser 1 is comprised of a paper material sheet comprising a main body centrally divided by a longitudinal folding line 3.

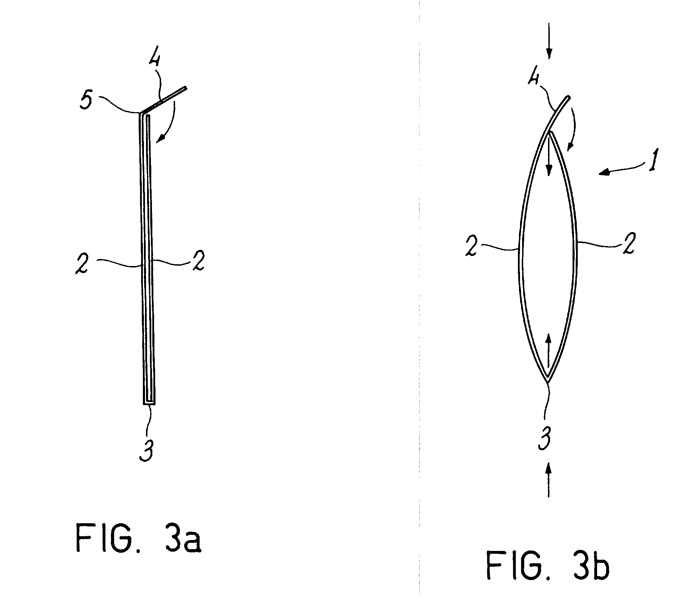

Laterally with respect to one of the half parts of the central body 2 a flap 4 for the closure of the canaliser 1 is coupled by a folding line 3 to obtain the shape shown in figures 3a and 3b (respectively the rest position and the ready to use position).

Each one of the two half parts of the central body 2 has the lateral edge having a first diverging length, along which said flap 4 is coupled, and a second converging length, in such a way that, when closed, has a substantially frustum conical shape in order to help the canalisation of the urine.

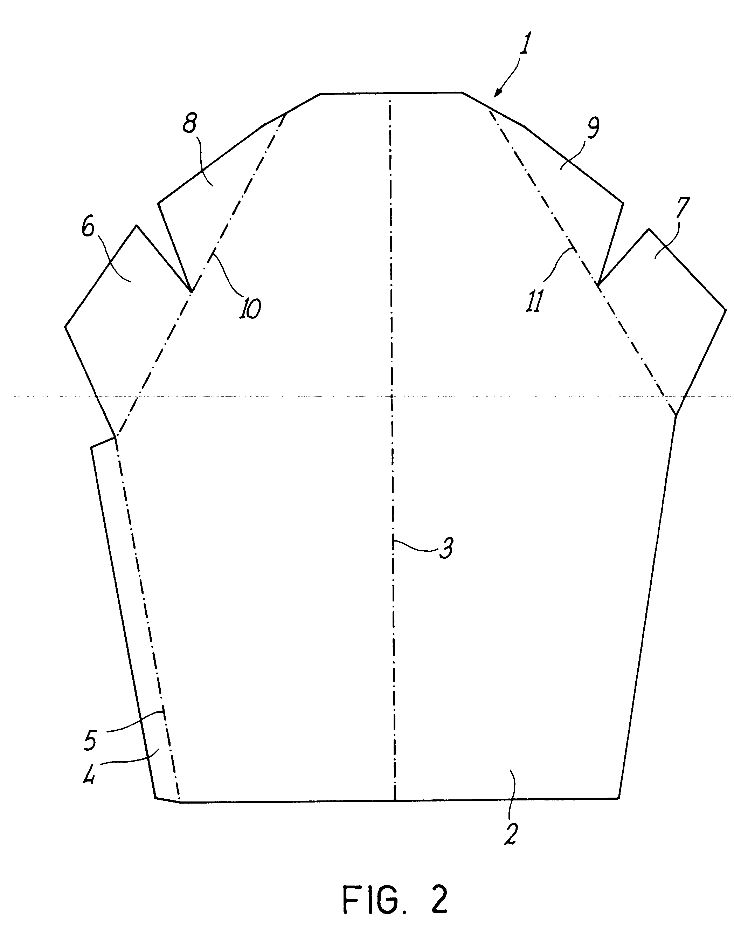

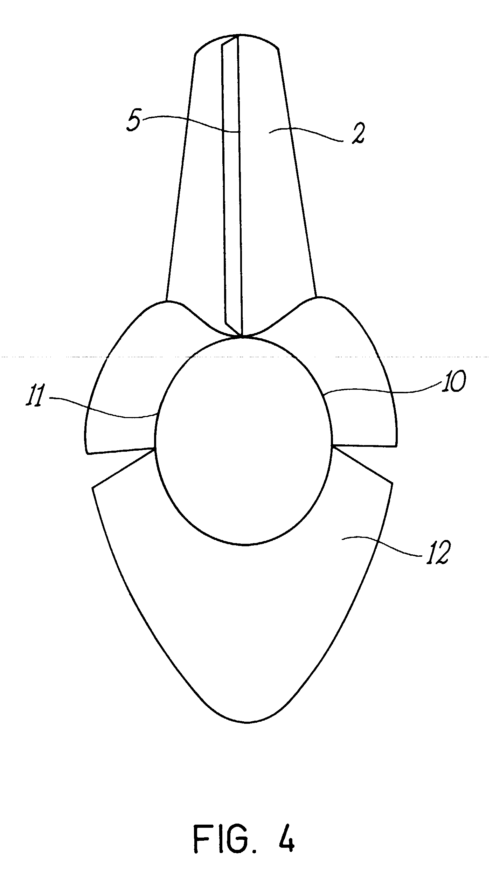

On the two diverging sides of the body 2 further pairs of flaps, respectively 6, 7 and 8, 9, can be provided, as shown in figure 2, said flap being foldable with respect to the body 2 along folding lines 10 and 11, for the coupling of a crown element (see figure 4).

Said crown element 12, innerly coated by soft and absorbing material has a double function:
1) it contains the urine within the concavity, making it flowing within the frustum conic element, absorbing possible leaking droplets, and
2) it works as "supporting frame" of the whole canaliser 1, when this is adhered to the vulva by two fingers of one hand (preferably the index and the digitus medius) that are placed like a fork laterally with respect to the "crown" and resting on the flaps, besides supporting the whole canaliser, determining the pressure necessary to make it adhering to terminate the urination.

Once the miction is terminated, the back portion of the crown, longer than its lateral parts (flaps) can be used, thank to its soft and absorbent, as well as sterile, coating, to completely dry the part.

The preparation of the canaliser 1 to the use provides the folding of the two half parts of the body 2 along the central line 3, bringing them in a parallel position each other.

A light pressure of the fingers squeezing the two half parts will be sufficient to create an ovalization of the structure (figure 3b) thus realising a frustum conical element.

The welding (gluing) of the flap 4 is not always necessary since many tests demonstrated that the canaliser 1 according to the invention works very well even if its upper portion is not coupled by welding (gluing).

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Sanitary canaliser to urinate, particularly for the female urination in public restrooms, characterized in that it is made up of a sheet element having a main body, foldable along a substantially central line and the lateral edges of which are shaped in such a way to make him assuming a substantially frusto conical shape when it is folded.

2. Sanitary canaliser according to claim 1, characterized in that on one of the lateral edges of said main body a closure foldable flap is provided.

3. Sanitary canaliser according to claim 1 or 2, characterized in that is made up of plasticized or waxed paper material.

4. Sanitary canaliser according to one of the preceding claims, characterized in that said lateral edges of said main body provide each one a first diverging length and a second converging length.

5. Sanitary canaliser according to one of the preceding claims, characterized in that on said lateral edges of the main body further flap for the coupling of a crown element are provided, said crown element being preferably comprised of soft, sterile and absorbing material.

6. Sanitary canaliser according to one of the preceding claims, characterized in that the inner part of the surface of the canaliser according to the invention is coated by a perfumed absorbing material.

7. Sanitary canaliser according to one of the preceding claims 1 - 5, characterized in that the inner part of the surface of the canaliser according to the invention is coated by a medicated material.

8. Sanitary canaliser according to one of the preceding claims, characterized in that said canaliser is comprised of two separated parts, the outer one of which can be used again and the inner one of which is soft and disposable.
